Europäisches Patentamt

European Patent Office

Office européen des brevets

(11) Numéro de publication: **0 231 668**
A1

## (12) DEMANDE DE BREVET EUROPEEN

(21) Numéro de dépôt: 86400122.7

(22) Date de dépôt: 22.01.86

(51) Int. Cl.4: **C12N 11/14** , C12N 9/26 , C12P 19/02 , C12P 19/14

(43) Date de publication de la demande:
12.08.87 Bulletin 87/33

(84) Etats contractants désignés:
AT BE CH DE GB IT LI LU NL SE

(71) Demandeur: **Groupement d'Intérêt Economique dit: SUCRE RECHERCHES ET DEVELOPPEMENTS**
**27-29, Rue Chateaubriand**
**F-75383 Paris Cédex 08(FR)**

(72) Inventeur: **Thibault, Philippe André**
**I.N.S.A. Départment Biochimique et Alimentaire**
**Avenue de Rangueil 31077 Toulouse Cedex(FR)**

(74) Mandataire: **de Boisse, Louis et al**
**CABINET DE BOISSE 37, Avenue Franklin D. Roosevelt**
**F-75008 Paris(FR)**

(54) **Enzyme invertase insolubilisée à haute activité spécifique, son procédé d'obtention et procédé utilisant cet enzyme.**

(57) L'invention se rapporte aux enzymes.

Elle concerne notamment une invertase insolubilisée fixée par une liaison covalente à un support solide, caractérisée en ce que l'invertase est fixée par covalence à du glutaraldéhyde qui est lié par covalence à un silane bifonctionnel lui-même fixé sur des particules poreuses de silice ayant une grosseur de particules comprise dans les gammes de 150 à 250 μm et un diamètre de pores compris dans la gamme de 10 à 30 nm, cette invertase insolubilisée ayant une activité spécifique d'au moins 6000 unités par gramme d'enzyme insolubilisé.

Utilisation pour l'hydrolyse du saccharose de sirops sucrés en glucose et fructose.

## Enzyme invertase insolubilisée à haute activité spécifique, son procédé d'obtention et procédé utilisant cet enzyme.

L'invention concerne une invertase insolubilisée à haute activité spécifique, un procédé d'obtention de cette invertase et un procédé de traitement de sirops sucrés utilisant cette invertase.

L'invertase est un enzyme bien connu provenant de la levure qui hydrolyse le saccharose en glucose et fructose.

Il a déjà été proposé (brevet français 2 472 015) d'immobiliser ou fixer l'invertase par une liaison covalente sur un support solide contenant de la cellulose et de la lignine, tel que des rafles de maïs, le processus de liaison faisant intervenir une oxydation ménagée, une amination par une diamine, une réduction et une activation, par exemple par du glutaraldéhyde, du support puis un couplage de l'enzyme au support ainsi pré-traité. Un tel composite d'invertase insolubilisée est utile pour traiter des jus sucrés de saccharose.

On connaît aussi, par le brevet français 2 078 426, des enzymes insolubilisés comprenant une matière siliceuse, un organosilane, tel qu'un $\gamma$-aminoalkyl-trialcoxysilane, lié à la matière siliceuse, un agent réticulant, tel que le glutaraldéhyde, lié par covalence à l'organosilane, et un enzyme lié par covalence à l'agent réticulant. Il n'est pas question dans ce brevet de l'invertase, ni de l'influence de la porosité ou de la grosseur des particules de la silice utilisée sur l'activité spécifique de l'enzyme insolubilisé. Ce brevet ne donne pas non plus beaucoup d'indications relativement aux conditions opératoires à observer pendant la préparation des enzymes insolubilisés.

Le brevet français 2 020 527 (correspondant au brevet U.S. N°3 519 538) décrit aussi des enzymes insolubilisés dans lesquels l'enzyme est couplé à un support par un agent de couplage bifonctionnel. Ce brevet ne parle pas de l'invertase, ni de l'influence de la porosité ou de la grosseur des particules de la silice utilisée sur l'activité spécifique de l'enzyme insolubilisé .

On connaît, par ailleurs, par le brevet français 2 223 324 de la Demanderesse, un procédé de fabrication de corps poreux à partir de divers oxydes, tels que la silice, ayant des diamètres de pores compris entre 10 et 100 nm, qui sont utiles comme support pour immobiliser ou fixer des enzymes. Il est dit dans ce brevet que le diamètre des pores doit être tel qu'il permette l'entrée de l'enzyme dans les pores. Toutefois, ce brevet ne parle pas de l'invertase, ni ne suggère l'existence d'une gamme de diamètres de pores pour laquelle l'invertase présenterait une activité spécifique optimale.

Bien que l'activité spécifique de l'invertase immobilisée du brevet français 2 472 015 soit relativement élevée, on a besoin dans l'industrie d'une invertase immobilisée encore plus active.

L'invention vise donc à fournir une telle invertase immobilisée à activité spécifique améliorée.

Plus particulièrement, l'invention concerne une invertase insolubilisée, fixée par covalence à du glutaraldéhyde qui est lié par covalence à un silane bifonctionnel lui-même fixé sur des partcules poreuses de silice ayant une grosseur de particules comprise dans la gamme de 150 à 250 µm, caractérisée en ce que les particules poreuses de silice ont un diamètre de pores compris dans la gamme de 10 à 30 nm, et en ce que cette invertase insolubilisée a une activité spécifique d'au moins 6000 unités par gramme d'enzyme insolubilisé.

Dans le présent mémoire, une unité d'activité spécifique correspond à 1 micromole de saccharose hydrolysé par minute dans les conditions suivantes :
température : 40°C
pH : 4,5
solution de saccharose à 100 g/litre.

L'activité spécifique de l'invertase insolubilisée de l'invention peut atteindre 10 000 unités/g.

L'invention concerne aussi un procédé de préparation de l'invertase insolubilisée de la revendication 1, comprenant les étapes consistant à :

a) traiter en présence d'eau des particules poreuses de silice ayant une grosseur de particules comprise dans la gamme de 150 à 250 µm par une solution d'un silane bifonctionnel ayant un groupe hydrolysable,

b) traiter le produit de l'étape (a) par une solution aqueuse de glutaraldéhyde, et

c) traiter le produit de l'étape (b) par une solution d'invertase, caractérisé en ce que :
-les particules poreuses de silice traitées dans l'étape (a), ont un diamètre de pores compris dans la gamme de 10 à 30 nm,
-dans l'étape (b) on utilise une solution aqueuse de glutaraldéhyde contenant au moins 2 % en poids/volume de glutaraldéhyde et on effectue le traitement, à un pH inférieur à 7, pendant au moins 1 heure environ, et
-dans l'étape (c), on utilise une solution d'invertase offrant une activité d'au moins 20 000 unités/gramme de silice et on effectue le traitement, à un pH de 5 à 6, pendant au moins 10 heures.

Comme silanes utilisables dans l'étape (a), on peut utiliser des silanes bifonctionnels ayant un groupe hydrolysable capable de se lier au support de silice et un autre groupe capable de réagir avec le glutaraldéhyde. Des exemples de silanes convenables sont donnés dans les brevets français 2 020 527 et 2 078 426 auxquels on se reportera pour plus de détails. L'influence de la nature du silane utilisé sur l'activité spécifique de l'enzyme insolubilisé est faible dans la mesure où l'enzyme n'est pas lié au silane mais sur le glutaraldéhyde, le rôle du silane se bornant donc à assurer le couplage du glutaraldéhyde au support de silice. En raison de leur disponibilité dans le commerce et des bons résultats obtenus avec eux, on préfère à ce jour utiliser des gamma-aminoalcoyltrialcoxysilanes et, en particulier, parmi eux, le gammaaminopropyltriéthoxysilane (appelé en abrégé A1100). L'invention, quoique non limitée à l'emploi du silane A1100, sera ci-après plus particulièrement décrite à propos de ce dernier.

L'étape de silanation (a) doit être conduite avec une concentration du silane A1100 d'au moins 4 % (poids/volume en abrégé p/v), car en-dessous de cette concentration minimale, on obtient une activité spécifique réduite. Il ne semble pas qu'il y ait de limite supérieure critique pour la concentration du silane, mais en pratique il y a peu d'avantages à tirer de l'emploi d'une concentration supérieure à 15 % (p/v) environ. Une gamme préférée de concentration du silane va de 5 à 10 % (p/v). Le pH doit être réglé dans la gamme de 4 à 5 pendant la silanation car en-dessous de 4, la cinétique de la réaction est trop lente et au-dessus de 5 il se produit une dégradation de la silice.

De préférence on utilise un pH égal à 4,5 ± 0,1. La température de silanation est aussi un paramètre important. Elle doit être d'au moins 70°C, de préférence d'au moins 75°C, et ne pas excéder 85°C. En-dessous de 70°C, on ne peut obtenir un enzyme immobilisé à haute activité spécifique et au-dessus de 85°C, on observe une dégradation des propriétés. Typiquement l'activité spécifique de l'enzyme insolubilisé sera d'autant plus grande que la température de silanation sera plus elevée dans l'intervalle de températures défini. La silanation doit être conduite pendant au moins 1 heure. Il n'y a pas de limite supérieure critique pour la durée de la silanation, mais en pratique, il y a peu d'avantages à tirer de durées de silanation excédant 2 heures.

L'étape (b) doit être conduite avec une concentration de glutaraldéhyde d'au moins 2 % (p/v) car des concentrations inférieures ne permettent pas d'obtenir la haute activité spécifique revendiquée. Il n'y a pas apparemment de limite supérieure de concentration critique. Toutefois, en pratique, il y a peu d'avantages à tirer de l'emploi de concentrations excédant 10 % (p/v). On préfère utiliser une solution de glutaraldéhyde comprise entre 2,5 et 5 % (p/v). Le pH doit être inférieur à 7 car au-dessus de ce pH, la cinétique de réaction est très ralentie. La température n'est pas particulièrement critique. On utilise commodément des températures ambiantes ou proches de celles-ci. La durée de l'étape (b) doit être d'au moins 1 heure pour l'btention d'un bon résultat. Il n'y a guère d'avantages a tirer de l'emploi de durées supérieures à 3 heures. On préfère une réaction de 1 à 2 heures environ.

L'étape (c) de fixation de l'invertase met en oeuvre une solution d'invertase libre devant offrir une activité d'au moins 20 000 U par gramme de support silice. Habituellement, l'activité offerte sera comprise entre 20 000 et 40 000 U/g de support bien qu'il n'y ait pas de limite maximale critique. Le pH est critique et doit être dans la gamme de 5 à 6 pour l'obtention de résultats convenables. On préfère un pH de 5 à 5,7 pour des résultats optimaux. La température n'est pas particulièrement critique. On utilise commodément des températures ambiantes ou proches de celles-ci.

La durée de l'étape de fixation doit être d'au moins 10 heures pour obtenir une bonne efficacité. En général, il y a peu d'avantages à tirer de prolonger la durée de fixation au-delà de 20 heures. On préfère des durées de 10 à 15 heures.

Bien entendu, dans les étapes (a), (b) et (c), il faut utiliser un excès de silane, de glutaraldéhyde et d'enzyme par rapport à la quantité à fixer sur le support solide. On a trouvé que, avec les concentrations prescrites, on obtenait de bons résultats en utilisant un rapport solution réactive/support de 10 ml de solution pour 1 g de support solide. Ce rapport n'est pas, toutefois, critique et on pourra utiliser des rapports solution réactive/support inférieurs ou supérieurs sans pour cela sortir du cadre de l'invention.

La grosseur des particules de silice et le diamètre des pores de celles-ci jouent un rôle essentiel. Il faut utiliser des particules poreuses d'une grosseur de 150 à 250 $\mu$m ayant un diamètre de pores compris dans la gamme de 10 à 30 nm, de préférence de 12 à 25 nm pour obtenir les meilleurs résultats. On a trouvé, en effet, que c'est ce type de particules qui se prêtent le mieux à production d'une invertase insolubilisée de haute activité spécifique.

L'invention concerne aussi un procédé de traitement d'un sirop sucré contenant du saccharose, caractérisé en ce qu'il consiste à mettre en contact le sirop et de l'invertase insolubilisée selon l'invention.

L'invertase insolubilisée de l'invention se prête particulièrement bien au traitement de sirops sucrés très concentrés, c'est-à-dire titrant 60 Brix et plus, qui habituellement sont difficiles à traiter par l'invertase en raison de leur relativement faible teneur en eau, ce qui affecte l'activité de l'enzyme.

La mise en contact du sirop et de l'enzyme insolubilisé peut se faire très simplement en faisant passer le sirop dans une colonne garnie de l'enzyme insolubilisé de l'invention.

Les exemples présentés ci-après sont donnés afin d'illustrer l'invention, sans la limiter.

Exemples 1 et 2 -Préparation d'une invertase insolubilisée très active.

A)En tube à essais:

Dans un tube à essais, on pèse 1 g de silice poreuse (diamètre moyen des pores : 25 nm - diamètre moyen des particules : 220 μ). On ajoute 10 ml d'une solution à 5 % (p/v) de γ-amino-propyltriéthoxy silane dans de l'eau distillée ajustée à pH 4,5 à l'aide d'acide acétique glacial.

Le tube, incliné, est fortement agité pendant 2 heures dans un bain-marie à 80°C.

Le produit obtenu est ensuite abondamment lavé à l'eau distillée afin d'éliminer le silane qui n'a pas réagi. On lui ajoute ensuite 10 ml d'une solution de glutaraldéhyde à 5 % (p/v) dans de l'eau distillée. Le pH, non ajusté, est de l'ordre de 3,5. On laisse agir deux heures à température ambiante, le tube étant placé horizontalement sur un agitateur à rouleaux, puis le composite est lavé plusieurs fois à l'eau distillée. Le dernier rinçage est effectué à l'aide de tampon acétate 0,1M, pH 5,25.

A partir d'une solution aqueuse glycérolée commerciale d'invertase pure de levure à 5000 U/ml, on prépare, par dilution dans du tampon acétate 0,2M, pH 5,25, une solution à 2000 U/ml. On ajoute au produit obtenu précédemment 10 ml de cette solution. Le tube est laissé 10 h à température ambiante sur agitateur à rouleaux. Le produit résultant est ensuite rincé plusieurs fois à l'eau distillée ; on le met en contact sous agitation 1 h à température ambiante avec 10 ml d'une solution de NaCl 1M puis on le rince avec du tampon acétate 0,1M, pH 5,25. Le produit enzyme insolubilisé obtenu présente une activité spécifique de 10 000 U/g environ.

B)En colonne :

Dans une colonne en verre de 60 ml de capacité et de 1,5 cm de diamètre, on place 10 g de silice poreuse (diamètre des pores 25 nm, diamètre moyen des particules : 220μ).

On prépare une solution à 5 % (p/v) de γ-amino-propyltriéthoxysilane ajustée à pH 4,5 (acide acétique glacial). On fait recirculer pendant 2 h à un débit d'environ 500 ml/h, 100 ml de cette solution dans la colonne maintenue à 80°C.

Après vidange, on lave le composite par circulation continue d'un demi-litre d'eau distillée (débit 500 ml/h). On prépare une solution de glutaraldéhyde à 5 % (p/v) dans de l'eau distillée (pH non ajusté) et on fait recirculer 100 ml de cette solution à température ambiante pendant deux heures, à raison d'un débit de 500 ml/h.

La colonne est ensuite vidangée et le composite rincé avec 500 ml d'eau distillée. On fait ensuite passer 100 ml de tampon acétate, 0,1M, pH 5,25 dans la colonne à un débit de 500 ml/h, puis on vidange.

A partir d'une solution aqueuse glycérolée commerciale d'invertase pure de levure à 5000 U/ml, on prépare par dilution dans du tampon acétate, 0,2M, pH 5,25, une solution à 2000 U/ml. On fait recirculer 100 ml de cette solution dans la colonne à température ambiante pendant 10 heures à un débit de 500 ml/h. La colonne est vidangée et le produit est rincé à l'eau distillée - (passage d'environ 1 l).

On fait recirculer pendant une heure (500 ml/h) 100 ml d'une solution molaire de NaCl, à température ambiante, puis on rince le produit résultant avec 500 ml de tampon acétate 0,1M, pH 5,25. Le produit enzyme insolubilisé obtenu a une activité spécifique d'environ 10 000 U/g.

Exemples 3 à 7 et exemple comparatif A

En suivant le mode opératoire 1A, on a préparé divers enzymes insolubilisés en utilisant des supports de silice ayant des grosseurs de pores différentes et en faisant varier divers paramètres. Les données et les résultats obtenus sont récapitulés au Tableau I ci-après.

TABLEAU I

| Exemples / Etapes | | A | 3 | 4 | 5 | 6 | 7 |
|---|---|---|---|---|---|---|---|
| Etape a | grosseur des pores de la silice, nm | 37,5 | 25,0 | 25,0 | 25,0 | 25,0 | 12,0 |
| | (3) concentration du silane, % (p/v) | 0,5 | 5 | 5 | 10 | 5 | 5 |
| | pH solution silane | 4,5 | 4,5 | 4,5 | 4,5 | 4,5 | 4,5 |
| | temps de silanation (°C) | 70 | 80 | 80 | 80 | 75 | 75 |
| | Durée de silanation (h) | 1 | 2 | 2 | 2 | 2 | 2 |
| Etape b | Solution de glutaraldéhyde (5%), Temp. ambiante, 2 heures de contact pH non ajusté ($\simeq$ 3,5) | | | | | | |
| Etape c | Solution d'invertase, U/g | 20000 | 20000 | 40000 | 20000 | 45000 | 45000 |
| | pH d'insolubilisation | 4,5 | 5,2 | 5,2 | 5,2 | 5,2 | 5,2 |
| | Temp. d'inso. (°C) | 30 | 30 | 30 | 30 | 30 | 30 |
| | Durée d'inso. (h) | 10 | 10 | 10 | 10 | 10 | 10 |
| | (1) Activité spécifique (U/g) | 500 | 7000 | 10000 | 7700 | 8000 | 7800 |
| | (2) Rendement de fixation (%) | 6 | 50 | 40 | 47 | 45 | 45 |

(1) dans les conditions standard
(2) c'est le rapport (X100) de l'activité spécifique sur la quantité d'Unités d'activité fixées
(3) $\gamma$-amino-propyltriéthoxysilane (A1100)

0 231 668

Exemples 8 à 10 et exemple comparatif B

Ces exemples illustrent l'emploi d'invertase insolubilisée pour hydrolyser le saccharose d'un sirop sucré concentré industriel glucose et fructose.

On a fait passer en continu un sirop sucré dans une colonne thermostatée d'une capacité de 60 cm³ environ contenant 5 g d'enzyme insolubilisé choisi parmi les enzymes des exemples A, 3 et 4, fonctionnant 24 h/24.

Les données et les résultats obtenus sont récapitulés au Tableau II ci-après.

On voit que les enzymes insolubilisés selon l'invention donnent une productivité très supérieure à celle obtenue avec un enzyme insolubilisé en dehors du cadre de l'invention.

## TABLEAU II

| Exemples / Conditions et résultats | B (enzyme de l'ex. A) | 8 (enzyme de l'ex. 3) | 9 (enzyme de l'ex. 3) | 10 (enzyme de l'ex. 4) |
|---|---|---|---|---|
| Température, °C | 50 | 50 | 40 | 50 |
| Activité spécifique de l'enzyme insolubilisé (U/g) | 500 | 7000 | 7000 | 7000 |
| Brix du sirop à 99,5% de pureté (%) | 67 | 67 | 60 | 67 |
| pH du sirop (ajusté à l'$H_3PO_4$ 85%) | 5,0 | 5,0 | 5,0 | 5,0 |
| Taux d'inversion (%) | 100 | 100 | 100 | 70 |
| Productivité (kg de saccharose hydrolysé/kg de composite x heures) | 0,2 | 3 | 4 | 6 |
| Stabilité | Pas de variation des performances sur 500h | Pas de variation des performances sur 500h | Pas de variation des performances sur 1200h | Pas de variation des performances sur 1500h |

## Revendications

1. Invertase insolubilisée, fixée par covalence à du glutaraldéhyde qui est lié par covalence à un silane bifonctionnel lui-même fixé sur des particules poreuses de silice ayant une grosseur de particules comprise dans la gamme de 150 à 250 μm,
caractérisée en ce que les particules poreuses de silice ont un diamétre de pores compris dans la gamme de 10 à 30 nm, et en ce que cette invertase insolubilisée à une activité spécifique d'au moins 6000 unités par gramme d'enzyme insolubilisé.

2. Invertase selon la revendication 1, caractérisé en ce qu'elle a une activité spécifique d'environ 10 000 unités g ou plus.

3. Invertase selon la revendication 1 ou 2, caractérisée en ce que les particules de silice ont une grosseur de pores comprise dans la gamme de 12 à 25 nm.

4. Procédé de préparation de l'invertase insolubilisée de la revendication 1, comprenant les étapes consistant à :

a) traiter en présence d'eau des particules poreuses de silice ayant une grosseur de particules comprise dans la gamme de 150 à 250 μm par une solution d'un silane bifonctionnel ayant un groupe hydrolysable,

b) traiter le produit de l'étape (a) par une solution aqueuse de glutaraldéhyde, et

c) traiter le produit le l'étape (b) par une solution d'invertase, caractérisé en ce que :
-les particules poreuses de silice traitées dans l'étape (a), ont un diamètre de pores compris dans la gamme de 10 à 30 nm,
-dans l'étape (b) on utilise une solution aqueuse de glutaraldéhyde contenant au moins 2 % en poids/volume de glutaraldéhyde et on effectue le traitement, à une pH inférieur à 7, pendant au moins 1 heure environ, et
-dans l'étape (c), on utilise une solution d'invertase offrant une activité d'au moins 20 000 unités/gramme de silice et on effectue le traitement, à un pH de 5 à 6, pendant au moins 10 heures.

5. Procédé selon la revendication 4, caractérisé en ce que le silane utilisé dans l'étape (a) est un gamma-aminoalcoyltrialcoxysilane.

6. Procédé selon la revendication 5, caractérisé en ce que le silane est du gamm-aminopropyltriéthoxysilane.

7. Procédé selon la revendication 5, caractérisé en ce que la concentration du silane est d'au moins 4% en poids/volume et le pH est compris dans la gamme de 4,4 à 4,6.

8. Procédé selon l'une quelconque des revendications 4 à 7, caractériseé en ce que la concentration de glutaraldéhyde est comprise dans la gamme de 2,5 à 5 % en poids/volume.

9. Procédé selon l'une quelconque des revendications 4 à 8, caractérisé en ce que dans l'étape (c), le pH est compris dans la gamme de 5 à 5,7.

10. Procédé de traitement d'un sirop sucré contenant du saccharose par de l'invertase insolubilisée pour convertir du saccharose en glucose et fructose, caractérisé en ce que l'invertase insolubilisée est telle que définie à l'une quelconque des revendications 1 à 3.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | BIOTECHNOLOGY AND BIOENGINEERING, vol. 26, no. 7, juillet 1984, pages 658-664, John Wiley & Sons Inc., New York, US; P. MONSAN et al.: "Invertase covalent grafting onto corn stover" * Page 658, colonne de droite, ligne 37 - page 659, colonne de gauche, ligne 2 * | 1,3-6, 10 | C 12 N  11/14 C 12 N   9/26 C 12 P  19/02 C 12 P  19/14 |
| | --- | | |
| Y | G. DURAND et al.: "Les enzymes production et utilisations industrielles", Biochimie appliquée, Collection dirigée par Claude Costes, Gauthier-Villars; * Page 110, tableau 6; page  110, paragraphe 1 * | 1,3,4, 10 | |
| | --- | | DOMAINES TECHNIQUES RECHERCHES (Int. Cl.4) |
| Y,D | US-A-3 669 841  (R.E. MILLER) * Colonne  2,  lignes  41,42; revendications  1-3,6,10,12-15,18 * & FR-A-2 078 426 | 1,4-6, 10 | C 12 N C 12 P |
| | ---                  -/- | | |

Le present rapport de recherche a été établi pour toutes les revendications

| Lieu de la recherche LA HAYE | Date d'achèvement de la recherche 19-09-1986 | Examinateur CHARLES D.J.P.I.G. |
|---|---|---|

| Catégorie | Citation du document avec indication, en cas de besoin, des parties pertinentes | Revendication concernée | CLASSEMENT DE LA DEMANDE (Int. Cl.4) |
|---|---|---|---|
| A | CHEMICAL ABSTRACTS, vol. 94, no. 5, février 1981, page 228, résumé no. 26791z, Columbus, Ohio, US; N.I. SHUSTROVA et al.: "Immobilization of yeast invertase on an inorganic carrier", & PRIKL. BIOKHIM. MIKROBIOL. 1980, 16(5), 780-3 | 1-9 | |
| | --- | | |
| A | APPLIED BIOCHEMISTRY & MICROBIOLOGY, vol. 14, no. 5, septembre/octobre 1978, pages 553-555, Plenum Publishing Corp., New York, US; T.A. LOGINOVA et al.: "Properties of proteases covalently immobilized on silica gel" * En entier * | 1-9 | |
| | --- | | DOMAINES TECHNIQUES RECHERCHES (Int. Cl.4) |
| A,D | US-A-3 519 538 (R.A. MESSING et al.) * Colonne 2, lignes 41-49; revendications 1-7,33-35 * | 1-9 | |
| | --- | | |
| A | CHEMICAL ABSTRACTS, vol. 90, no. 19, 7 mai 1979, page 240, résumé no. 147795e, Columbus, Ohio, US; & SU-A-644 760 (ALL-UNION SCIENTIFIC-RESEARCH BIOTECHNICAL INSTITUTE) 30-01-1979 * Résumé * | 1 | |
| | --- -/- | | |

Le present rapport de recherche a été établi pour toutes les revendications

| Lieu de la recherche | Date d'achèvement de la recherche | Examinateur |
|---|---|---|
| LA HAYE | 19-09-1986 | CHARLES D.J.P.I.G. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille. document correspondant

OEB Form 1503 03 82

## DOCUMENTS CONSIDERES COMME PERTINENTS

Page   3

| Catégorie | Citation du document avec indication, en cas de besoin, des parties pertinentes | Revendication concernée | CLASSEMENT DE LA DEMANDE (Int. Cl.4) |
|---|---|---|---|
| A,D | FR-A-2 223 324  (CORNING GLASS WORKS) <br> * Page 3, lignes 5-32 * <br> --- | 1 | |
| A,D | WO-A-8 101 860  (BEGHIN-SAY SA) <br><br> & FR-A-2 472 015 <br><br> ----- | | |

DOMAINES TECHNIQUES
RECHERCHES (Int. Cl.4)

Le present rapport de recherche a été etabli pour toutes les revendications

| Lieu de la recherche | Date d'achèvement de la recherche | Examinateur |
|---|---|---|
| LA HAYE | 19-09-1986 | CHARLES D.J.P.I.G. |